(19) Europäisches Patentamt
European Patent Office
Office européen des brevets

(11) Publication number: **0 277 292 B1**

(12) # EUROPEAN PATENT SPECIFICATION

(45) Date of publication of patent specification: **08.05.91** (51) Int. Cl.$^5$: **C07F 9/547**

(21) Application number: **87116893.6**

(22) Date of filing: **16.11.87**

(54) **Organic solvent-free process for preparing pyrimidinyl organophosphates.**

(30) Priority: **22.12.86 US 945258**

(43) Date of publication of application:
**10.08.88 Bulletin 88/32**

(45) Publication of the grant of the patent:
**08.05.91 Bulletin 91/19**

(84) Designated Contracting States:
**BE CH DE ES FR GB IT LI NL**

(56) References cited:
**DE-A- 2 753 455**
**GB-A- 2 011 415**
**NL-A- 6 809 749**

(73) Proprietor: **THE DOW CHEMICAL COMPANY**
**2030 Dow Center Abbott Road P.O. Box 1967**
**Midland Michigan 48640-1967(US)**

(72) Inventor: **Johnson, Mark R.**
**1070 Borden**
**Roselle Illinois 60172(US)**

(74) Representative: **Huber, Bernhard, Dipl.-Chem. et al**
**Möhlstrasse 22 Postfach 860 820**
**W-8000 München 86(DE)**

**EP 0 277 292 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Pyrimidinyl organophosphorous compounds, such as those described in U.S. Patents 2,754,243, 4,429,125 and 4,127,652, are useful as insecticides for controlling insects such as corn rootworm, codling moth, thrips and leafhoppers. The latter two patents describe processes for making these compounds, by contacting the requisite pyrimidinol, potassium carbonate, acetonitrile and halo-phosphoroester reactants. Similarly, U.S. Patents 4,007,197 and 4,147,866 describe processes for preparing the pyrimidinyl phosphates by contacting the requisite pyrimidinol and phosphoroester reactants in the presence of a catalyst in an inert organic liquid reaction medium under alkaline conditions. Great Britain patent specification 2,011,415 describes a process wherein hydroxypyrimidine is converted into a sodium salt with sodium hydroxide under reflux and in the presence of a hydrocarbon solvent and a phase transfer catalyst. Water is continuously removed by azeotropic distillation and the dried reactant is contacted with an aliphatic thiophosphoric halide. This reference also teaches that the presence of water in the reaction mixture causes lower yields and by-products formation.

The above processes employ either a 1-phase or a 2-phase mixture of either a water-immiscible or a water-miscible organic solvent with or without water being present. While such processes generally achieve good yields, they have the disadvantage of requiring the use of organic solvents which must be removed at the completion of the reaction. This separation of the organic solvent requires the use of multiple steps and high energy requirements. After separation, the organic solvents require either waste disposal and/or recycling procedures using elaborate and expensive recycling apparatus. Further the very presence of an organic solvent gives rise to the formation of undesirable by-products. NL-A-6809749 disclosed a process for the preparation of 0,0-dialkyl-0-arylphosporothioates from 0,0-dialkylchloro-thiophosphates and phenolates in aqueous solution at a temperature between 20° C and 100° C under addition of a water insoluble tertiary amine catalyst, e.g. heterocyclic nitrogen bases.

The present invention provides a process for preparing a pyrimidinyl organophosphorous compound of the formula

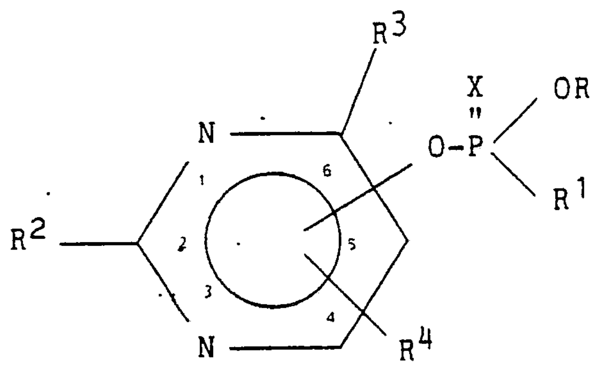

(I)

in which

R is alkyl of 1 to 6 carbon atoms;

$R^1$ is alkyl of 1 to 6 carbon atoms, alkoxy of 1 to 6 carbon atoms, alkylthio or monoalkylamino of 1 to 6 carbon atoms;

$R^2$ is hydrogen, alkyl of 1 to 8 carbon atoms, alkenyl of 1 to 4 carbon atoms, alkylthiomethyl, alkylsulfinylmethyl or alkylsulfonylmethyl whose alkyl is of 1 to 4 carbon atoms, phenyl, phenylthio, alkylthio, alkylsulfinyl or alkylsulfonyl whose alkyl is of 1 to 4 carbon atoms, or alkylthioethylthio wherein alkyl is of 1 to 2 carbon atoms;

$R^3$ is hydrogen or alkyl of 1 to 3 carbon atoms;

$R^4$ is hydrogen, alkyl of 1 to 4 carbon atoms, or alkylthio of 1 to 2 carbon atoms; and

X is oxygen or sulphur;

comprising contacting an aqueous solution containing a pyrimidinate of the formula

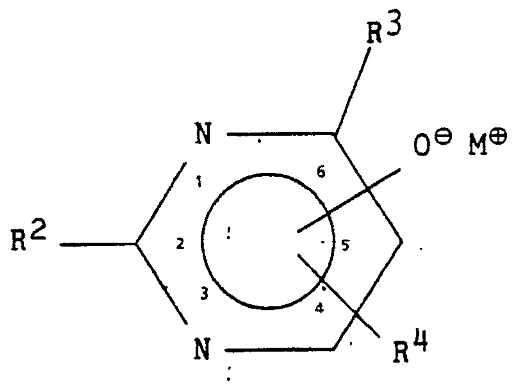

(II)

wherein $M^{\oplus}$ represents an alkali, alkaline earth metal or ammonium, with a phosphoroester of the formula

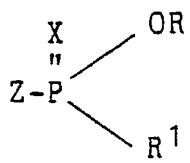

(III)

in which
R, $R^1$, $R^2$, $R^3$, $R^4$ and X have the above-mentioned meanings; and
Z represents halogen, preferably chlorine, or tosylate
in the presence of a phase transfer catalyst and in the absence of an added organic solvent. The pyrimidinyl organophosphorous compound can be recovered by conventional means.

Preferably $R^2$ is t-butyl, $R^3$ is hydrogen, $R^4$ is hydrogen in the 4-position on the pyrimidinyl ring, and the $-O^{\ominus}M^{\oplus}$ is on the 5-position on the ring. Also preferred is that $M^{\oplus}$ is the sodium salt. Preferably Z is halogen, most preferably chloro, X is sulfur, R is ethyl and $R^1$ is ethoxy ($-OC_2H_5$). Also preferred is that $R^1$ is isopropoxy ($-OC(CH_3)_2$). Also preferred is that $R^2$ is isopropyl ($-CH(CH_3)_2$), $R^3$ is methyl, $O^{\ominus}M^{\oplus}$ is in the 4-ring position and $R^4$ is hydrogen in the 5-ring position, a compound commonly known is diazinon. Preferably the phase transfer catalyst is a quaternary ammonium salt, most preferably tetrabutyl ammonium bromide (TBAB).

The process of the present invention has the advantage of being above to utilize a mixture containing alkali, alkaline earth metal or ammonium pyrimidinate salt and water without having to add an organic solvent. Another advantage of the present invention is that it simplifies the preparation of pyrimidinyl organophosphorous compounds by reducing the number of steps necessary for their preparation. And still yet another advantage of the present invention is that it reduces or eliminates the formation of undesirable by-products otherwise present in known processes which utilize organic solvents.

The term "alkyl" is used in the present specification and in the appended claims to designate a straight or branched saturated hydrocarbon moiety (i.e., hydrocarbons having carbon-carbon single bonds) such as, for example, methyl, ethyl, propyl, isopropyl, butyl, isobutyl, t-butyl, pentyl and hexyl..

The term "alkoxy" is employed herein to mean an alkoxy moiety having a straight or branched saturated hydrocarbon moiety such as, for example, methoxy, ethoxy, propoxy, isopropoxy, butoxy and pentoxy.

The term "phase transfer catalyst" is intended to mean a material which catalyzes a reaction by the transfer of one phase to another. Phase transfer catalysts suitable for carrying out the process of the present invention include the quaternary ammonium and phosphonium salts, crown ethers, kryptates, glycol ethers and certain tertiary amines, including 4-dimethyl aminopyridine.

The quaternary ammonium and phosphonium salts suitable as catalysts include essentially any compound from the known class of quaternary ammonium and phosphonium salts. Suitable quaternary ammonium and phosphonium salts have a minimum solubility of at least 1 weight percent in the liquid reaction medium at 25°C and normally have a total aggregate carbon content of at least 4 carbon atoms and preferably from 12 to 31 carbon atoms. The ammonium and phosphonium salts can be represented by

the formula $R_1'R_2'R_3'R_4'Q^+A^-$, wherein $R_1'$-$R_4'$ are hydrocarbyl groups (e.g. alkyl, aryl, alkaryl, aralkyl or cycloalkyl) and $Q^+$ is a quaternized atom of nitrogen or phosphorous. Additionally, $R_1'$ can join with $R_2'$ to form a 5- or 6-membered heterocyclic compound having at least one quaternized nitrogen or phosphorous atom in the ring and may also contain one non-adjacent atom of nitrogen, oxygen or sulfur within the ring. Typically, $R_1'$-$R_2'$ are hydrocarbyl groups of from 1 to 12 carbon atoms. $A^-$ is an inert neutralizing anion and may be varied to convenience. Chloride and bromide are the preferred anions but other suitable anions include, for example, fluoride, iodide, bisulfate, hydroxide, acetate, tosylate and benzoate. The following compounds are illustrative: tetraalkyl ammonium salts, such as tetra-n-butyl, tetrahexyl-, tri-n-butylmethyl-, cetyltrimethyl-, trioctylmethyl-and tridecylmethyl ammonium chlorides, bromides, bisulfates and tosylates; aralkylammonium salts, such as tetrabenzylammonium chloride, benzyltrimethyl-, benzyltriethyl-, benzyltributyl-, and phenethyltrimethylammonium chlorides and bromides; arylammonium salts, such as triphenylmethylammonium fluoride, chloride or bromide, N,N,N-trimethylanilinium bromide, N,N-diethyl-N-methylanilinium bisulfate, trimethylnaphthylammonium chloride, p-methylphenyltrimethylammonium chloride or tosylate; 5- and 6-membered heterocyclic compounds containing at least one quaternized nitrogen atom in the ring, such as N-methylpyridinium chloride or methyl sulfate, N-hexyl pyridinium iodide, (4-pyridyl)-trimethylammonium chloride, 1-methyl-1-azabicyclo[2.2.1]heptane bromide, N,N-dibutylmorpholinium chloride, N-ethylthiazolium chloride and N-butylpyrrolium chlorides and the corresponding phosphonium salts.

The ammonium salts are currently preferred over the phosphonium salts due to cost and commercial availability. The preferred catalysts are benzyltrimethyl-, benzyltriethyl-, tetra-n-butyl and tri-n-butylmethyl ammonium salts, most preferably tetra-n-butyl.

The phase transfer catalysts are used in the process in small but catalytic amounts. For example, amounts from 0.1 to 20 mole percent, based on the reactants, are suitable but amounts of from 0.1 to 10 mole percent are generally preferred.

A base or buffer is used to prepare or to maintain the pyrimidinate reactant in its anionic or salt form before or during its reaction with the phosphoroester. The base or buffer can comprise, for example, an alkali (Na, K, Li) or alkaline (Ca, Mg) earth metal, ammonium ($NH_4$) or any suitable countercation; and an anion suitable for providing the proper pH such as, for example, hydroxide (OH), bicarbonate ($HCO_3$), carbonate ($CO_3$), phosphate ($PO_4$) hydrogen phosphates ($HPO_4$), dihydrogen phosphate ($H_2PO_4$), or borate ($BO_3$).

The amount of base or buffer employed in the present process can range from 1 to 1.5 moles per one mole of pyrimidinol, preferably about 1.1:1 (moles base or buffer:moles pyrimidinol).

The alkali metal pyrimidinates of Formula (II) can be contacted with the halophosphoroester of Formula (III) in molar ratios ranging from 0.95:1 to 1.5:1, more preferably 1:1 (alkali metal pyrimidinate:phosphoroesters).

The contacting of the alkali metal pyrimidinate and the phosphoroester is carried out at temperatures ranging from ambient to 120°C, preferably from 50° to 80°C. The contacting is normally carried out at ambient pressures with stirring or other means of agitation.

The amount of water in association with the alkali metal pyrimidinate, is such that the alkali metal pyrimidinate concentration in water can range from 5 percent to 60 percent or the upper limit of solubility for the pyrimidinate salt, preferably from 15 to 30 percent. The aqueous solution containing the reactants may contain crude products or impurities which do not adversely affect the reaction between the alkali pyrimidinate and the phosphoroester. Such materials can include by-products from the in situ preparation of the pyrimidinate starting materials, which contain sodium chloride or sodium bromide.

In situations where the phosphoroester contains sulfur (i.e., X = sulfur), the process can be conducted under aerobic conditions (i.e., in the presence of air) although, it is preferred that the reaction be maintained under anaerobic conditions in order to minimize formation of the oxygen analogue. Such anaerobic conditions can be maintained with purging or blanketing with an inert gas, such as nitrogen or helium.

After the reaction is completed, the desired organopyrimidinyl phosphorous compound of Formula I is recovered by conventional recovery procedures such as phase separation. Any catalyst residues and inorganic salts associated in or with the aqueous solution are typically removed along with the aqueous solution by, for example, a simple wash, ion-exchange treatment, filtration, washing, distillation or recrystallization.

The following examples further illustrate the present invention.

Example 1 - Preparation of 0,0-diethyl 0-(2-(1,1-dimethylethyl)-5-pyrimidinyl)phosphorothioate

Step A

2-t-Butyl-5-bromopyrimidine (98.0 g, 0.46 mol), sodium methoxide (295 g of 25 percent solution, 1.37 mol), and cuprous chloride (0.90 g, 0.01 mol) were placed into a 1-liter Hastalloy-C Parr reactor and heated to 105 degrees Centigrade (°C) for 6 hours, then to 155°C for 14 hours. After cooling and venting the dimethyl ether vapors, the slurry was diluted with 535 g of water and filtered through diatomaceous earth, on a glass frit. The filtrate was extracted with 258 g of xylenes to remove trace organics, the layers were separated, and the aqueous layer was neutralized to a pH of 10.2 with 33 g of concentrated HCl. The resulting solution containing crude sodium 2-t-butyl-5-pyrimidinate was concentrated to approximately 35 percent of the original solution weight by removal of methanol/water by flash distillation at atmospheric pressure. Analysis of the concentrated solution by high pressure liquid chromatography (HPLC) indicated it contained 21.4 percent by weight sodium 2-t-butyl-5-pyrimidinate, representing a 92.4 percent yield based on bromopyrimidine. This solution of sodium 2-t-butyl-5-pyrimidinate is used directly for thiophosphorylation reactions.

Step B

A 250 ml 3-necked flask equipped with air-driven stirrer and condenser was charged with 81.3 g (0.100 M) of the crude sodium 2-t-butyl-5-pyrimidinate solution as described in Step A above, sparged with nitrogen for 15 minutes, and treated with 18.7 g (0.099 M) of diethylchlorothiophosphate (DEPCT) and 0.55 g (1.00 mmol) of 59 percent aqueous tetrabutylammonium bromide solution. The mixture was stirred and heated to 60°C for 1.5 hours, at which time the pH was adjusted from 7.1 to 10.7 by addition of 50 percent NaOH. The solution was stirred and heated another 40 minutes, and then allowed to cool to room temperature. The brine phase was removed, the organic phase was washed once with 50 ml of 1 N HCl, and dried under reduced pressure to yield 28.3 g of 0,0-diethyl 0-(2-(1,1-dimethylethyl)-5-pyrimidinyl)-phosphorothioate of 97.9 percent assay (91 percent yield).

Example 2 - Preparation of 0,0-diethyl 0-(2-(1,1-dimethylethyl)-5-pyrimidinyl)phosphorothioate

A 250 ml 3-necked flask equipped with condenser and air-driven stirrer was charged with 7.44 g of 50 percent sodium hydroxide, and 65 ml of pH = 9 buffer solution. This solution was sparged with nitrogen for 20 minutes and then treated with 15.2 g of 2-t-butyl-5-pyrimidinol and 0.55 g of 60 percent tetrabutyl ammonium bromide. When the pyrimidinol was completely dissolved, 18.9 g of diethylchlorothiophosphate was added. The solution was stirred at 60°C for 45 minutes, treated with 0.50 g 50 percent sodium hydroxide, stirred another 45 minutes at 60°C, treated with an additional 0.22 g 50 percent sodium hydroxide, and stirred another 3.5 hours at 60°C. The mixture was then allowed to cool, the phases separated, the organic phase was washed with 50 ml of 1 N HCl, and dried under vacuum to yield 28.3 g of 0,0-diethyl 0-(2-(1,1-dimethylethyl)-5-pyrimidinyl)phosphorothioate of 93 percent yield of product assay greater than 97 percent.

Example 3 - Preparation of 0,0-diethyl 0-(2-(1,1-dimethylethyl)-5-pyrimidinyl)phosphorothioate

The above procedure described in Example 2 was employed, except that 2.44 g of 1 percent 4-dimethylaminopyridine (DMAP) was substituted for tetrabutylammonium bromide, and the 50 percent NaOH additions were modified. After 2 hours reaction time, 1.0 g of 50 percent NaOH was added, and after 3.5 hours, 0.65 g 50 percent NaOH was added. Product yield of 0,0-diethyl 0-(2-(1,1-dimethylethyl)-5-pyrimidinyl)phosphorothioate of 93 percent yield of product assay of greater than 97 percent.

Example 4 - Preparation of 0,0-diethyl 0-(2-(1,1-dimethylethyl)-5-pyrimidinyl)phosphorothioate

A solution of 15.2 g of 2-t-butyl-5-pyrimidinol, 7.6 g of 50 percent sodium hydroxide, and 0.55 g of 59 percent tetrabutyl ammonium bromide (TBAB) in 65 ml of pH 9 buffer was treated with 20.0 g ethylisopropylchlorothiophosphate and heated to 70°C with stirring. The pH of the reaction mixture was maintained above 9 by periodic addition of small amounts of 50 percent sodium hydroxide. After 4 hours

the mixture was allowed to cool, the phases separated, and the organic layer washed once with 50 ml of 1 N HCl. The product was dried under reduced pressure to yield 27.2 g of 0,0-diethyl 0-(2-(1,1-dimethylethyl)-5-pyrimidinyl)phosphorothioate of assay greater than 97 percent.

## Claims

1. A process for preparing a pyrimidinyl organophosphorous compound of the formula

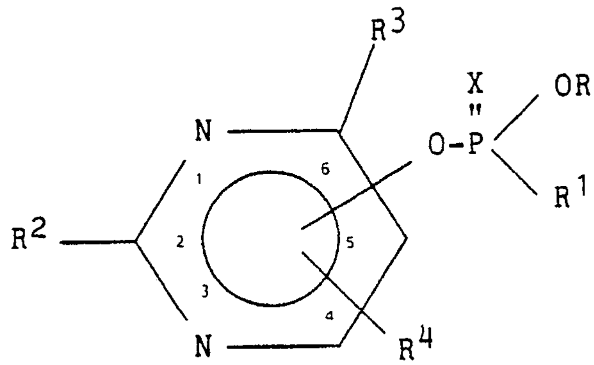

(I)

in which
R is alkyl of 1 to 6 carbon atoms;
$R^1$ is alkyl of 1 to 6 carbon atoms, alkoxy of 1 to 6 carbon atoms, alkylthio or monoalkylamino of 1 to 6 carbon atoms;
$R^2$ is hydrogen, alkyl of 1 to 8 carbon atoms, alkenyl of 1 to 4 carbon atoms, alkylthiomethyl, alkysulfinylmethyl or alkylsulfonylmethyl whose alkyl is of 1 to 4 carbon atoms, phenyl, phenylthio, alkylthio, alkylsulfinyl or alkylsulfonyl whose alkyl is of 1 to 4 carbon atoms, or alkylthioethylthio wherein alkyl is of 1 to 2 carbon atoms;
$R^3$ is hydrogen or alkyl or 1 to 3 carbon atoms;
$R^4$ is hydrogen, alkyl of 1 to 4 carbon atoms, or alkylthio of 1 to 2 carbon atoms; and
X is oxygen or sulphur;
comprising contacting an aqueous solution containing a pyrimidinate of the formula

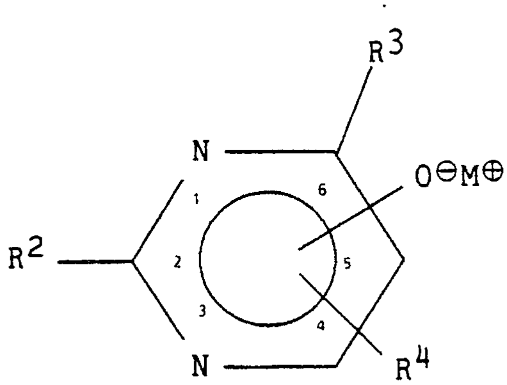

(II)

wherein $M^{\oplus}$ represents an alkali, alkaline earth metal or ammonium, with a phosphoroester of the formula

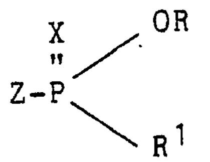

(III)

in which
R, $R^1$, $R^2$, $R^3$, $R^4$ and X have the above-mentioned meanings; and
z represents halogen, preferably chlorine, or tosylate
in the presence of a quaternary ammonium salt as a phase transfer catalyst and in the absence of an added organic solvent.

2. The process of Claim 1 wherein $R^2$ is t-butyl, and $R^3$ and $R^4$ are hydrogen.

3. The process of Claim 1 wherein $M^{\oplus}$ is sodium or potassium.

4. The process of any one of Claims 1 to 3 wherein Z is halogen, X is sulfur, R is ethyl and $R^1$ is ethoxy.

5. The process of any one of Claims 1 to 3 wherein Z is halogen, X is sulfur, R is ethyl and $R^1$ is isopropoxy.

6. The process of any one of Claims 1 to 5 wherein the pyrimidinate concentration in the aqueous solution ranges from 5 to 60 percent by weight.

7. The process of Claim 6 wherein the pyrimidinate concentration is 15 to 30 percent by weight.

## Revendications

1. Procédé de préparation d'un composé organo-phosphoré à groupe pyrimidinyle, de formule

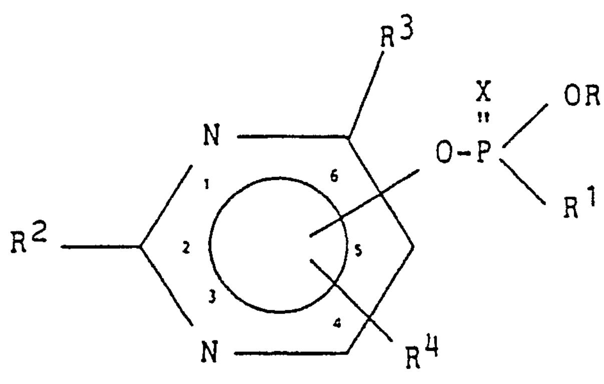

(I)

dans laquelle
R est un groupe alkyle comportant 1 à 6 atomes de carbone,
$R^1$ est un groupe alkyle comportant 1 à 6 atomes de carbone, alcoxy comportant 1 à 6 atomes de carbone, alkylthio ou monoalkylamino comportant 1 à 6 atomes de carbone,
$R^2$ est un atome d'hydrogène, un groupe alkyle comportant 1 à 8 atomes de carbone, alcényle comportant 1 à 4 atomes de carbone, alkylthiométhyle, alkylsulfinylméthyle ou alkylsulfonylméthyle, dont le groupe alkyle comporte 1 à 4 atomes de carbone, phényle, phénylthio, alkylthio, alkylsulfinyle ou alkylsulfonyle, dont le groupe alkyle comporte 1 à 4 atomes de carbone, ou alkylthioéthylthio dont le groupe alkyle comporte 1 à 2 atomes de carbone,
$R^3$ est l'hydrogène ou un groupe alkyle comportant 1 à 3 atomes de carbone,
$R^4$ est l'hydrogène, un groupe alkyle comportant 1 à 4 atomes de carbone, ou alkylthio comportant 1 à

2 atomes de carbone, et
X est l'oxygène ou le soufre,
comprenant la mise en contact d'une solution aqueuse contenant un pyrimidinolate de formule :

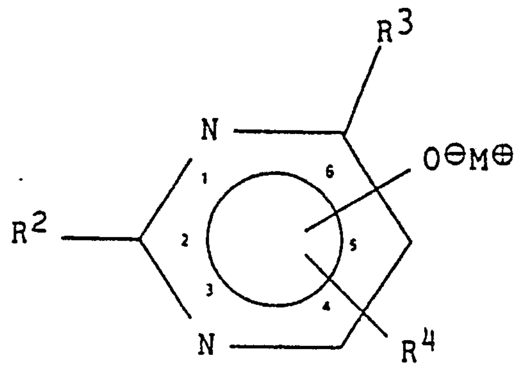

(II)

dans laquelle $M^{\oplus}$ représente un métal alcalin, alcalino-terreux ou l'ammonium, avec un ester phosphoré de formule

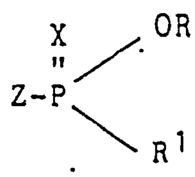

(III)

formules dans lesquelles
R, $R^1$, $R^2$, $R^3$, $R^4$ et X ont les significations mentionnées cidessus, et
Z représente un halogène, de préférence le chlore, ou un tosylate,
en présence d'un sel d'ammonium quaternaire comme catalyseur de transfert de phase et en l'absence d'un solvant organique ajouté.

2. Procédé selon la revendication 1, dans lequel $R^2$ est le groupe t-butyle et $R^3$ et $R^4$ sont l'hydrogène.

3. Procédé selon la revendication 1, dans lequel $M^{\oplus}$ est le sodium ou le potassium.

4. Procédé selon l'une quelconque des revendications 1 à 3, dans lequel Z est un halogène, X est le soufre, R est le groupe éthyle et $R^1$ est un groupe éthoxy.

5. Procédé selon l'une quelconque des revendications 1 à 3, dans lequel Z est un halogène, X est le soufre, R est le groupe éthyle et $R^1$ est le groupe isopropoxy.

6. Procédé selon l'une quelconque des revendications 1 à 5, dans lequel la concentration en pyrimidinolate de la solution aqueuse est comprise entre 5 et 60% en poids.

7. Procédé selon la revendication 6, dans lequel la concentration en pyrimidinolate est de 15 à 30 % en poids.

**Ansprüche**

1. Verfahren zur Herstellung einer Pyrimidinyl-Organo-phosphor-Verbindung der Formel

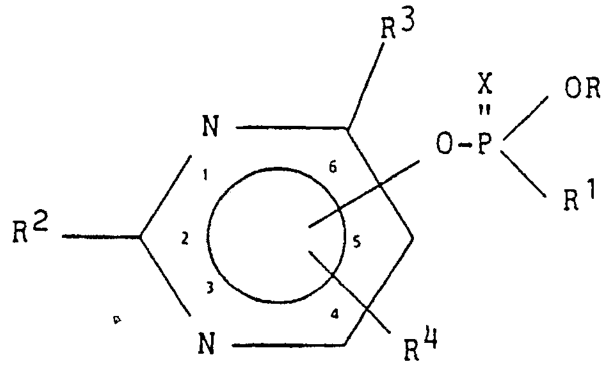

(I)

worin R Alkyl von 1 bis 6 Kohlenstoffatomen ist;
$R^1$ Alkyl von 1 bis 6 Kohlenstoffatomen, Alkoxy von 1 bis 6 Kohlenstoffatomen, Alkylthio oder Monoalkylamino von 1 bis 6 Kohlenstoffatomen ist;
$R^2$ Wasserstoff, Alkyl von 1 bis 8 Kohlenstoffatomen, Alkenyl von 1 bis 4 Kohlenstoffatomen, Alkylthiomethyl, Alkylsulfinylmethyl oder Alkylsulfonylmethyl, deren Alkyl von 1 bis 4 Kohlenstoffatomen ist, Phenyl, Phenylthio, Alkylthio, Alkylsulfinyl oder Alkylsulfonyl, deren Alkyl von 1 bis 4 Kohlenstoffatomen ist, oder Alkylthioethylthio, wobei Alkyl von 1 bis 2 Kohlenstoffatomen ist;
$R^3$ Wasserstoff oder Alkyl von 1 bis 3 Kohlenstoffatomen ist;
$R^4$ Wasserstoff, Alkyl von 1 bis 4 Kohlenstoffatomen oder Alkylthio von 1 bis 2 Kohlenstoffatomen ist; und
X Sauerstoff oder Schwefel ist;
umfassend das Kontaktieren einer wäßrigen Lösung, enthaltend ein Pyrimidinat der Formel

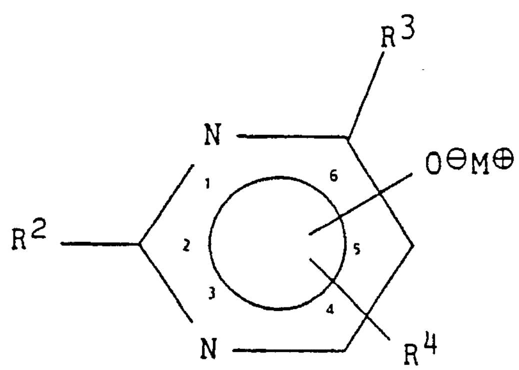

(II)

worin $M^{\oplus}$ ein Alkali-, Erdalkalimetall oder Ammonium darstellt, mit einem Phosphorester der Formel

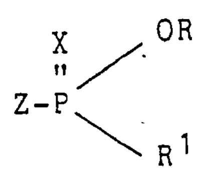

(III)

worin
R, $R^1$, $R^2$, $R^3$, $R^4$ und X die oben erwähnten Bedeutungen besitzen; und
Z Halogen, vorzugsweise Chlor, oder Tosylat darstellt,
in der Gegenwart eines quaternären Ammoniumsalzes als Phasentransferkatalysator und in der Abwesenheit eines zugegebenen organischen Lösugsmittels.

2. Verfahren nach Anspruch 1, worin $R^2$ t.-Butyl ist, und $R^3$ und $R^4$ Wasserstoff sind.

3. Verfahren nach Anspruch 1, worin $M^{\oplus}$ Natrium oder Kalium ist.

4. Verfahren nach einem der Ansprüche 1 bis 3, worin Z Halogen, X Schwefel, R Ethyl und $R^1$ Ethoxy ist.

5. Verfahren nach einem der Ansprüche 1 bis 3, worin Z Halogen, X Schwefel, R Ethyl und $R^1$ Isopropoxy ist.

6. Verfahren nach einem der Ansprüche 1 bis 5, worin die Pyrimidinat-Konzentration in der wäßrigen Lösung von 5 bis 60 Gew.-% reicht.

7. Verfahren nach Anspruch 6, worin die PyrimidinatKonzentration von 15 bis 30 Gew.-% ist.